# EUROPEAN PATENT APPLICATION

(11) **EP 2 476 760 A1**
(43) Date of publication of application: **18.07.2012**
(21) Application number: 10815433.7
(22) Date of filing: 09.09.2010
(51) Int. Cl.: C12Q 1/68, C12N 15/09

(54) **METHOD FOR ANALYZING NUCLEIC ACID MUTATION USING ARRAY COMPARATIVE GENOMIC HYBRIDIZATION TECHNIQUE**

(30) Priority: 10.09.2009 JP 2009209578
(71) Applicant: FUJIFILM Corporation, Tokyo 106-0031 (JP)
(72) Inventor: KURAMITSU, Masayuki, Ashigarakami-gun Kanagawa (JP); IWAKI, Yoshihide, Ashigarakami-gun Kanagawa (JP); UJIHARA, Dai, Ashigarakami-gun Kanagawa (JP)
(74) Representative: HOFFMANN EITLE
(86) International application number: PCT/JP2010/065562
(87) International publication number: WO 2011/030838

(57) **Abstract**

There is provided a method for analyzing nucleic acid mutation using an array comparative genomic hybridization technique, which reduces the false positive and the false negative and improves the reliability of the analysis results.

A method for analyzing nucleic acid mutation using array comparative genomic hybridization technique comprising:
[(a) a step of bringing a plurality of labeled sample nucleic acids one by one into contact with the plural same probe nucleic acid sets to effect hybridization], [(b) a step of obtaining label intensities F1 to Fn of the sample nucleic acids having been hybridized in each spot of the spots X1 to Xn to which the same probe nucleic acid has been immobilized], [(c) a step of determining whether each of n-1 pieces of comparison values from a comparison value of F1 with F2 to a comparison value of F1 with Fn falls within a prescribed numerical value range or not], and
[(d) a step of comparing whether the number of the comparison values having been determine as not falling within the prescribed numerical value range exceeds a prescribed number or not and, in the case of exceeding the prescribed number, judging the spot X1 positive]. n is an integer of 3 or more.

## Description

### Technical Field

The present invention relates to a method for analyzing nucleic acid mutation using an array comparative genomic hybridization technique, which reduces influences of false positive and the like from analysis results and improves the reliability of the analysis results.

### Background Art

An array comparative genomic hybridization (CGH) technique is known as a method for detecting genome structure disorder such as genome copy number disorder occurring at a several 10 kb to several Mb level for a short period of time (see, e.g., Non-Patent Documents 1 and 2).
In the case of a diagnosis array for genetic diseases and the like using array CGH, it becomes necessary to accurately diagnose the copy number disorder which directly connects to a pathological condition. Therefore, a nucleic acid as a standard necessarily has no copy number variation (CNV) and thus careful selection and confirmation including the case of rare CNV should be performed, so that there frequently exist cases which are troublesome and require re-inspection.
Moreover, in the case where the copy number disorder is detected including arrays for whole genome analysis, since it is very difficult to obtain a standard nucleic acid which has been proved to be normal with regard to a target genome, it is necessary to perform inspection of parents and siblings continuously and to perform re-inspection repeatedly for confirming whether the case is false positive or false negative or de novo copy number disorder together with verification with analysis examples on normal analytes and public databases (see, e.g., Non-Patent Document 1). In the case where it is judged false positive or false negative, re-inspection becomes necessary and there arises a problem that diagnosis cannot be made with one-time inspection.

### Background Art Documents

### Non-Patent Documents

Non-Patent Document 1: "Arei CGH Shindan Katsuyo Gaido Bukku Shitte Okitai Senshokutai Bisaikozo Ijosho (Arrey CGH Diagnosis Utilization Guidebook, Chromosome Fine Structure Disorder Which Is Need To Know)" edited by Joji Inazawa et al., Iyaku(Medicine and Drug) Journal Co., Ltd.
Non-Patent Document 2: Issei Imoto, Joji Inazawa "Maikuro Arei Gijusu wo Mochiita CGH Kaiseki: CGH Arei Hou (CGH Analysis Using Microarray Technology: CGH Array Method)", Saibo Kogaku (Cell Technology), Vol. 23, No. 03, 355-361 (2004).

### Summary of the Invention

### Problems that the Invention is to Solve

In view of the above problems of the background art, an object of the present invention is to provide a method for analyzing nucleic acid mutation which reduces influences of the false positive and the false negative in analysis results, particularly, influences owing to CNV of a standard nucleic acid, which are problems in conventional array comparative genome hybridization techniques, and improves the reliability of the analysis results without needing re-inspection as well as a program for performing the data processing.
Moreover, another object of the invention is to provide a method for diagnosing a disease derived from gene mutation using the method for analyzing nucleic acid mutation.

### Means for Solving the Problems

The above problems have been solved by the following methods.
1. A method for analyzing nucleic acid mutation using array comparative genomic hybridization technique comprising:
   (a) a step of, using n sets of the same probe nucleic acid sets where a plurality of spots are present on one nucleic acid microarray and probe nucleic acids different from one another have been immobilized on a plurality of the spots, bringing n kinds of labeled sample nucleic acids S1 to Sn one by one into contact with n sets of the probe nucleic acid sets to effect hybridization,
   (b) a step of selecting n pieces of spots X1 to Xn to which the same probe nucleic acid has been immobilized, which are selected from n sets of the probe nucleic acid sets, and obtaining label intensities F1 to Fn of the sample nucleic acid having been hybridized in n pieces of the respective spots X1 to Xn,
   (c) a step of comparing the label value F1 of the spot X1 with each of the label values F2 to Fn to thereby obtain n-1 pieces of comparison values C2 to Cn and determining whether each of the comparison values falls within a prescribed numerical value range or not, and
   (d) a step of determining whether the number of the comparison values having been determined as not falling within the prescribed numerical value range in the step (c) among n-1 pieces of the comparison values exceeds a prescribed number or not and, in the case of exceeding the prescribed number, judging the spot X1 positive,
   n being an integer of 3 or more, the sample nucleic acid being an analyte nucleic acid or a standard nucleic acid, at least the S1 being an analyte nucleic acid, and X1 being a spot with which S1 having been brought into contact.
2. The method for analyzing nucleic acid mutation according to 1 above, wherein at least two kinds among n kinds of the sample nucleic acids are analyte nucleic acids.
3. The method for analyzing nucleic acid mutation according to 1 or 2 above, which further comprises:
   (c') a step of determining whether each of n-1 pieces the comparison values from a comparison value of F2 with F1 to a comparison value F2 with Fn falls within a prescribed numerical value range or not and
   (d') a step of determining whether the number of the comparison values having been determined as not falling within the prescribed numerical value range in the step (c') among n-1 pieces of the comparison values exceeds a prescribed number or not and, in the case of exceeding the prescribed number, judging the spot X2 positive, the spot X2 being a spot with which a sample nucleic acid S2 different from the above S1 having been brought into contact.
4. The method for analyzing nucleic acid mutation according to any one of 1 to 3 above, wherein at least one kind among n kinds of the sample nucleic acids is a standard nucleic acid.
5. The method for analyzing nucleic acid mutation according to any one of 1 to 3 above, wherein n kinds of the sample nucleic acids are all analyte nucleic acids.
6. The method for analyzing nucleic acid mutation according to any one of 1 to 5 above, wherein n kinds of the sample nucleic acids are nucleic acids derived from different individuals belonging to taxonomically the same species.
7. The method for analyzing nucleic acid mutation according to any one of 1 to 6 above, wherein
   n pieces of the same probe nucleic acid sets NS1 to NSn are provided,
   each probe nucleic acid set NSi is provided on a nucleic acid microarray having p pieces of spots tᵢₗ to tᵢₚ, i being an integer and satisfying 1≤i≤n and p being an integer and satisfying 2≤p,
   probe nucleic acids different from one another are immobilized on p pieces of the spots tᵢₗ to tᵢₚ of each probe nucleic acid set NSi.
   one of p pieces of the spots of each probe nucleic acid set NSi is the spot Xi, the same probe nucleic acid is immobilized on the spots tᵢⱼ to tₙⱼ, j being an integer and satisfying 1≤j≤p,
   the step (a) comprises bringing the labeled sample nucleic acid Si into contact with the probe nucleic acid set NSi to hybridize the sample nucleic acid Si to the nucleic acids having been immobilized on p pieces of the spots of the probe nucleic acid set NSi,
   the step (b) comprises obtaining label values Fᵢₗ to Fᵢₚ of the sample nucleic acid having been hybridized in the spots tᵢₗ to tᵢₚ of each probe nucleic acid set NSi,
   NS1 and NSm are selected from the probe nucleic acid sets NS1 to NSn and m being an integer and satisfying 2≤m≤n,
   the step (c) comprises the steps:
   (c1) comparing the label values Flj and Fmj to determine p pieces of comparison values Cl" to Cp",
   (c2) calculating an average value or a central value of the comparison values Cl" to Cp", and
   (c3) setting a prescribed numerical value range based on the average value or central value obtained in the step (c2) and judging whether the comparison values Cl" to Cp" fall within the prescribed numerical value range or not.
8. The method for analyzing nucleic acid mutation according to any one of 1 to 7 above, wherein the sample nucleic acids are all labeled with the same label.
9. The method for analyzing nucleic acid mutation according to any one of 1 to 8 above, wherein the label intensities F1 to Fn in the step (b) are corrected values.
10. A program for performing data processing in the method for analyzing nucleic acid mutation according to any one of 1 to 9 above, which performs the following procedures (I) and (II):
   [(I) a procedure of comparing whether n-1 pieces of comparison values from F1 with F2 to F1 with Fn fall within a prescribed numerical value range or not]
   [(II) a procedure of judging the spot X1 positive in the case where, among all of n-1 pieces of the comparison values temporarily decided in the above (I), the number of the comparison values having been determined as not falling within the prescribed numerical value range by the procedure (I) are present with exceeding a prescribed number].
11. The program according to 10 above, wherein the procedure (i) includes the following procedures (i) to (v):
   [(i) a procedure of, in p pieces of the spots present in each of n sets of the probe nucleic acid sets,
      selecting a pair of two probe nucleic acid sets to be selected so as to contain the spot X1 from n sets of the probe nucleic acid sets and
      calculating a comparison value of the label intensities of paired spots to which the same probe nucleic acid has been immobilized, between the two probe nucleic acid sets]
   [(ii) a procedure of performing the calculation of the comparison value in the procedure (i) on each of p pieces of paired spots in the probe nucleic acid sets],
   [(iii) a procedure of calculating an average value or a central value of p pieces of the comparison values obtained in the procedure (ii)], and
   [(iv) a procedure of setting a prescribed numerical value range from the average value or central value obtained in the procedure (iii) and comparing whether each of p pieces of the comparison values exceeds the prescribed numerical value or not]
12. A method for diagnosing a disease derived from gene mutation, which uses the method for analyzing nucleic acid mutation according to any one of 1 to 9 above.

### Advantage of the Invention

The method for analyzing nucleic acid mutation according to the present invention can reduce influences of false positive and false negative in analysis results, particularly, influences owing to CNV of a standard nucleic acid, which are problems in conventional array comparative genome hybridization techniques, and can improve the reliability of the analysis results. More specifically, even for the analytes which need re-inspection by the influences of false positive and false negative in conventional array comparative genome hybridization techniques, the method for analyzing nucleic acid mutation according to the present invention does not require the re-inspection.
Since the program of the invention performs data processing in the above method for analyzing nucleic acid mutation, the influences of false positive and false negative in analysis results, particularly the influences owing to CNV can be reduced and the reliability of the analysis results can be improved.
Since the method for diagnosing a disease derived from gene mutation according to the invention includes using the above method for analyzing nucleic acid mutation, diagnosis can be made with reducing the influences of false positive and false negative.

### Brief Description of the Drawings

[FIG. 1] FIG. 1 is a flow chart showing the method for analyzing nucleic acid mutation of one embodiment of the invention.
[FIG. 2] FIG. 2 is a partial schematic view showing a part of one embodiment of the method for analyzing nucleic acid mutation of one embodiment of the invention.
[FIG. 3] FIG. 3 is a drawing showing a result of logarithmic plots of fluorescence intensity ratios of analyte DNA No. 1 to male genomic DNA as a standard.
[FIG. 4] FIG. 4 is a drawing showing a result of logarithmic plots of fluorescence intensity ratios of analyte DNA No. 1 to analyte DNA No. 2.
[FIG. 5] FIG. 5 is a drawing showing a result of logarithmic plots of fluorescence intensity ratios of analyte DNA No. 1 to analyte DNA No. 3.
[FIG. 6] FIG. 6 is a drawing showing a result of logarithmic plots of fluorescence intensity ratios of analyte DNA No. 1 to analyte DNA No. 4.

### Mode for Carrying Out the Invention

In the present description, "to" representing a range shows a range including the numerical values described before and after the word as a minimum value and a maximum value, respectively.
The method for analyzing nucleic acid mutation according to the present embodiment is a method for analysis using an array comparative genomic hybridization technique, which includes the following steps (a) to (d).

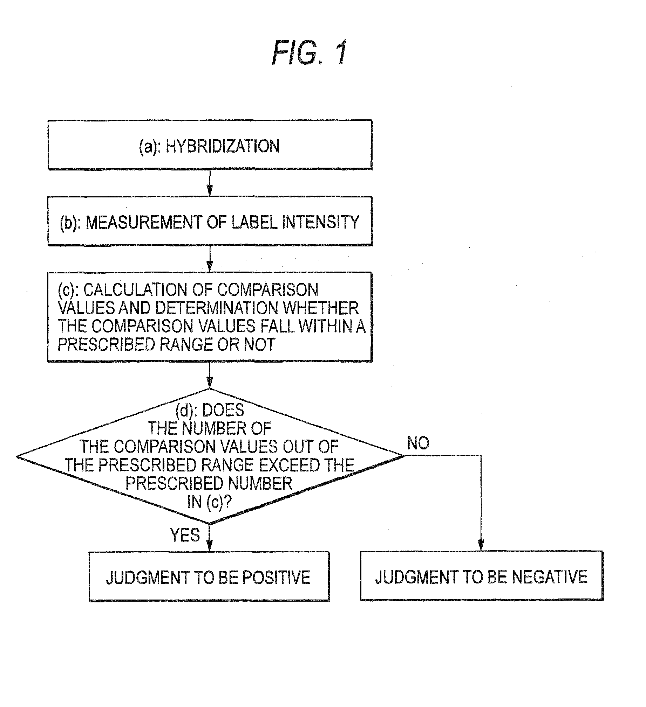

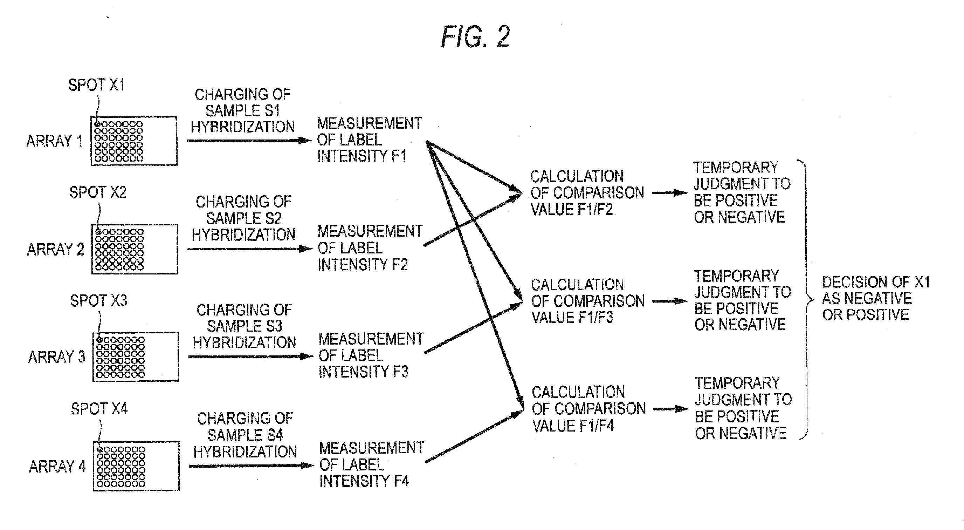

FIG. 1 and FIG. 2 are schematic views showing one preferable embodiment of the method for analyzing nucleic acid mutation according to the invention. In this regard, the number of spots to be a judging criterion in the step (d) is made half in FIG. 1 and a case where n is 4 is exemplified in FIG. 2 but the present embodiment is not limited thereto.
First, in the step (a), using n sets of the same probe nucleic acid sets where probe nucleic acids different from one another have been immobilized to p or more pieces of the spots on a nucleic acid microarray, n kinds of sample nucleic acids of S1 to Sn are one by one brought into contact with the probe nucleic acid sets to effect hybridization. Hereinafter, p is an integer of 2 or more and n is an integer of 3 or more.
Then, n pieces of spots X1 to Xn to which the same probe nucleic acid has been immobilized, which are selected from each of n sets of the probe nucleic acid sets, are selected and label intensities F1 to Fn of the sample nucleic acid having been hybridized in respective spots (label intensity of the spot X1: F1, label intensity of the spot X2: F2 ... label intensity of the spot Xn: Fn) are obtained (step (b)).
Next, it is determined whether each of n- 1 pieces of the comparison values from a comparison value of F2 with F1 to a comparison value F1 with Fn falls within a prescribed numerical value range or not (step (c)). In FIG. 2, the comparison value C2 of F1 with F2 is shown as F1/F2 in the step 3 and the comparison value C3 of F1 with F3 is shown as F1/F3, but the calculation method of the comparison value is not limited thereto as mentioned below.
Then, when the number of the comparison values having been determined as not falling within the prescribed numerical value range in the step (c) among n-1 pieces of the comparison values exceeds a prescribed number, the spot X1 is decided as positive (step (d)).

Incidentally, explanation is conducted here with focusing an arbitrary spot X1 but can be conducted with replacing the spot by the other spot. It is preferable to perform the analysis with replacing the above X1 by all the spots in the array to which the sample nucleic acid S1 has been hybridized.

Here, the array comparative genomic hybridization technique (hereinafter sometimes also referred to as array CGH technique) means a technique where a comparative genomic hybridization technique is performed on a nucleic acid microarray. For example, the technique has been precisely described in "Arei CGH Shindan Katsuyo Gaido Bukku Shitte Okitai Senshokutai Bisaikozo Ijosho" edited by Joji Inazawa et al., (Iyaku(Medicine and Drug) Journal Co., Ltd) and the like.

In the conventional array CGH technique, there is known a technique using a two-color method where different kinds of labeling compounds are used in a standard nucleic acid and in an analyte and are simultaneously hybridized into the same spot.
In the two-color method, the standard and the analyte are simultaneously evaluated in the same array but, in the case where CNV of the standard nucleic acid is unknown, it is difficult to determine whether the increase or decrease appearing as a result of measurement is an increase or decrease in the standard or an increase or decrease in the influence of the analyte (so-called false positive/false negative). In order to avoid such a problem, in the two-color method, it is conducted to confirm CNV of the standard beforehand. However, in the case where CNV of the standard is unknown, many samples and experiments have been needed for evaluation of CNV of the standard.
On the other hand, in the method of the present embodiment, a nucleic acid as an analyte and one nucleic acid as a control are each hybridized in different spots to which the same kind of a nucleic acid has been immobilized and measured values are compared with each other. However, even in this method, when a nucleic acid of an analyte is added to an array 1, a nucleic acid of an analyte as a control is added to an array 2, each is hybridized, label intensities in the spot X1 and the spot X2 that are spots to which the same probe nucleic acid has been immobilized are compared to thereby obtain one comparison value, and judgment for positive or negative is done based on it, particularly in the case where the control contains CNV (copy number variation), an increase or decrease by the influence of the control (so-called false positive/false negative) is generated and thus there is a case where re-inspection is needed or a case where misjudgment occurs.
Therefore, in the present embodiment, n kinds of labeled sample nucleic acids are used. n is an integer of 3 or more. Specifically, one kind is focused as a judging target among n kinds of the sample nucleic acids, n-1 pieces of comparison values with regard to the judgment target are obtained using remaining n-1 kinds as controls, and temporary judgment for positive or negative of the comparison values is performed. Then, among n-1 pieces of results of the temporary judgment, a case where a prescribed number or more of the results are temporary positive is judged true positive and a case where the number of the results judged temporary positive is less than the prescribed number is judged true negative.
As the prescribed number, it is preferably to use a number in the range of 30% to 74% of the total number of the results of the temporary judgment and a number of 40% to 60% is more preferably used.
Here, positive/negative is the same as in the conventional array CGH technique and a case where the nucleic acid sequence having been immobilized to the target spot is present in larger amount in the verifying nucleic acid as compared with the standard nucleic acid or a case where present in smaller amount or a case where not present is called positive and a case where the amount is equal is called negative.

In general, in a certain spot, a probability of generating false positive and false negative (hereinafter simply referred to as false positive and the like) in the comparison of the analyte nucleic acid with one kind of the control nucleic acid is at most about 1/10, usually 1/100 or less. Accordingly, n-1 kinds of the control nucleic acids are used and, among resulting n- 1 pieces of results of the temporary judgment, a case where a prescribed number or more of the results are temporary positive is judged true positive, thereby the probability of generating the above false positive being reduced to a large extent.
As above, false positive and the like can be reduced from the measurement results by the method for analyzing nucleic acid mutation according to the present embodiment.
Moreover, since the rate of the above generation becomes extremely low in the present embodiment, it becomes possible to use no standard nucleic acid.

The kind n of the sample nucleic acids to be used in the present embodiment is 3 or more and is preferably 4 or more.

As mentioned above, the effect is high as n increases and an upper limit of n is not limited but, in order to obtain data rapidly or from the viewpoint of simplifying the calculation, n is preferably 100 or less. It is most preferable that n is 4 to 100.

Hereinafter, the labeled n kinds of the sample nucleic acids are also shown as sample nucleic acids S1, S2, ... Sn-1, Sn (n is an integer of 3 or more).
Hereinafter, with focusing the spot (X1), explanation is performed as verification whether the label in the spot (X1) is positive or not, namely as verification on the presence or absence of nucleic acid mutation (S1) in the labeled sample nucleic acid brought into contact with the spot (X1) but the spot (X1) is an arbitrary spot. Usually, the method of the embodiment is conducted with focusing each spot of p pieces or more of spots (p is an integer of 2 or more) on an array and preferably, verification is conducted with focusing all the spots on the array, to which the sample nucleic acids have been hybridized.
Moreover, among the sample nucleic acids, a focused nucleic acid is used as a nucleic acid to be judged and a counter one to be compared is used as a control nucleic acid but any of the sample nucleic acids can be focused and can be used as a nucleic acid to be judged. It is preferable that at least two kinds of the sample nucleic acids are analyte nucleic acids. Namely, the above nucleic acid as S1 can be arbitrarily selected from n kinds of the nucleic acids and a highly reliable analysis of nucleic acid mutation can be conducted by carrying out the present embodiment with using all the analyte nucleic acids contained in n kinds of the nucleic acids as S1 sequentially.
By comparing the label intensities of corresponding spots with regard to the sample nucleic acids, true positive or negative for plural analyte nucleic acids can be judged.
Moreover, the step of performing hybridization and the step of obtaining the label intensities are usually carried out once for each of all the spots on the array.
In this regard, it is also possible to perform verification by the method of the embodiment with regard to the data in which the label intensities after hybridization have been previously obtained.

### Step (a)

In the step (a), n kinds of labeled sample nucleic acids of S 1 to Sn are one by one brought into contact with n pieces of spots X1 to Xn to which the same probe nucleic acid has been immobilized to thereby hybridize the sample nucleic acids to the above immobilized nucleic acids.

The nucleic acid microarray means one where a probe nucleic acid is bound on a solid substrate in a high density. The solid material for use in the above solid substrate is not particularly limited as long as it is a material to which a probe nucleic acid can be bound, for example, a glass material such as a slide glass commonly used and also a plastic material.

As the nucleic acid microarray to be used in the present embodiment, a BAC-DNA array, an oligonucleotide microarray, a c-DNA microarray, and the like may be mentioned.

In the method for analyzing nucleic acid mutation according to the embodiment, a commercially available nucleic acid microarray may be used or one prepared by a usual method may be used.

The spot means a region to which a probe nucleic acid has been immobilized in a large amount in a high density and the plural spots are present on the above nucleic acid microarray.
The number of the above spots on the nucleic acid microarray is not particularly limited as long as the number is p pieces or more but generally, the number of 5 pieces to 1,000,000 pieces is preferable and the number of 300 pieces to 5,000 pieces is preferable.
The arrangement of the spots on the above nucleic acid microarray is not particularly limited and there may be mentioned a multi-analyte array having an array in plural areas and capable of measuring plural sample nucleic acids on one substrate, a tie ring array where plural arrays are combined and a large number of spots are reproduced on one substrate, and the like.

The probe nucleic acid means a nucleic acid which has been immobilized on the substrate of the nucleic acid microarray (preferably plural pieces in the form of spots) and is preferably a nucleic acid containing a known sequence.
The size of the probe nucleic acid is preferably 10 bases (b) to 10 kb, more preferably 50 b to 5 kb, and further preferably 100 b to 2 kb.
"The same probe nucleic acid" also includes a nucleic acid which hybridizes to a nucleic acid complimentary to the above probe nucleic acid under stringent conditions.

In the present embodiment, hybridization is performed with different sample nucleic acids in n pieces of the spots to which the same probe nucleic acid has been immobilized, respectively. Here, the spots to which the same probe nucleic acid has been immobilized are represented as spots X1, X2, ... Xn-1, Xn (n is an integer of 3 or more).

The n pieces of the spots X1, X2, ... Xn-1, Xn may be present on the same nucleic acid microarray but are preferably present on n pieces of different nucleic acid microarrays. For example, in the case where n pieces of the spots are present on n pieces of the same kinds of nucleic acid microarrays separately, it is preferred that spots having the same block number, column number, or raw number are used as n pieces of the above spots.
The amount of the probe nucleic acid to be immobilized is preferably 0.8 to 5 µg, more preferably 1.0 to 2.0 µg per spot.

In the step (a), the "probe nucleic acid set" means a group of probe nucleic acids coding a gene for a specific transcription product. In the present embodiment, the number of the probe nucleic acid sets usually corresponds the number of spots on the array.

For one probe nucleic acid set, one kind of the probe nucleic acid may be immobilized or the plural probe nucleic acids may be immobilized on each spot as long as the kind of the probe nucleic acid to be immobilized is different among individual spots. Usually, one probe nucleic acid set is immobilized on one array.
In the step (a), n sets of the probe nucleic acid sets mean n sets of the same probe nucleic acid sets and also may include a probe nucleic acid set which hybridizes to a probe nucleic acid set complimentary to a certain probe nucleic acid set under stringent conditions.
The above same probe nucleic acid set corresponds to the kinds of the sample nucleic acids and is n sets.

As the above probe nucleic acid, there may be mentioned chemically synthesized nucleic acids, cDNA, BAC (Bacterial Artificial Chromosomes), YAC (Yeast Artificial Chromosomes), PAC (P1-derived Artificial Chromosomes), those obtained by amplifying them using a genetic engineering technique.
Furthermore, a nucleic acid obtained by chemical modification of a natural nucleic acid such as DNA and RNA may be used as the probe nucleic acid. For example, PNA (Peptide Nucleic Acid), BNA (Bridged Nucleic Acid), methyl phosphonate-type DNA, phosphorothioate-type DNA, phosphoramidate-type DNA, boranophosphate-type DNA, and the like can be used. Moreover, a chimera-type nucleic acid can be also employed.
As methods for binding the probe nucleic acid on a solid substrate, there may be mentioned a method of chemically synthesizing nucleotides one by one using an amidite monomer to which a photo-eliminating group represented by Gene Chip has been bound and using a mask or a method of spotting an amidite monomer on the solid substrate by an inkjet method to chemically synthesize a desired sequence, a method of synthesizing a nucleic acid on the substrate by changing pH of the solution on an electrode and eliminating a protective group with utilizing the pH change, a method of purifying a previously chemically synthesized nucleic acid and spotting it on the substrate, a method of spotting cDNA using a spotter, and the like. As the spotting methods, an inkjet method, a pin array method, and the like may be mentioned.

The sample nucleic acid means a single-strand or double-strand nucleic acid to be used in the method for analyzing nucleic acid mutation according to the present embodiment and may be any of DNA, RNA, and the like.

The origin of the sample nucleic acid is not particularly limited and, for example, there may be mentioned analytical targets such as cells containing sample nucleic acid for which expressed amount and existing amount of nucleic acid, copy number in genome, and the like are to be measured using a nucleic acid microarray or cells for which evaluation such as diagnosis is to be performed, and also tissues, organs, one individual, and the like, and the origin is not limited to a cell itself.
Particularly, the origin of the sample nucleic acid is preferably a cell or the like obtainable from a human patient, an animal, or the like which is considered to have a certain disease. As the disease, there may be included all diseases such as cancers, genetic diseases, and infectious diseases for which a nucleic acid may have some information, and the disease is not particularly limited. The method of the present embodiment is suitable for inspections for congenital genetic diseases in which the number of mutation is relatively small. Noninvasive sites such as mucous membranes, hairs, and nails and body fluids such as blood, lymph, bone marrow, semen, and amniotic fluid are preferably used as samples.
The n kinds of the sample nucleic acids are preferably nucleic acids derived from different individuals belonging to taxonomically the same species or nucleic acids derived from different cells of the same individual, more preferably nucleic acids derived from human different individuals or nucleic acids derived from different cells (e.g., normal cell and cancer cell) of human the same individual. Nucleic acids derived from human different individuals are most preferred.
Moreover, the method of the present embodiment is preferably used in the inspection for individuals not selected depending on the possibility or the like of having a specific genetic disease (one hundred percent inspection of newborn babies or the like).

At preparation (e.g., purification, fragmentation) of the sample nucleic acid, it is preferable to remove cell-dissolved matter such as proteins and lipids by a purification treatment. This is because various side reactions occur at labeling with fluorescence or the like and also the cell-dissolved matter such as proteins and lipids largely influence background noise when purification is not conducted, so that performance of the nucleic acid microarray is remarkably lowered.

As methods for the purification, there may be mentioned a method of using a cartridge supported with a nucleic acid-adsorbable membrane of silica, cellulose compound, or the like, precipitation with ethanol or precipitation with isopropanol, extraction with phenolchloroform, and the like. Furthermore, there may be mentioned methods with solid-phase extraction cartridge, chromatography, and the like using ion-exchange resins, silica supports bonded with a hydrophobic substituent such as an octadecyl group, resins having a size-exclusion effect. The length of the sample nucleic acid is not particularly limited and, for example, may be a whole genome but the above purification treatment may be conducted after the following fragmentation treatment.
In this regard, the length of the labeled sample nucleic acid is not particularly limited but the nucleic acid is usually fragmented to 1,000 bases (1 kb) or base pairs (bp) to 5,000 b or bp by a labeling/fragmentation treatment.

As the fragmentation treatment, an enzymatic treatment, an ultrasonic treatment, a mechanical crushing treatment, a chemical treatment, and the like may be mentioned. As an enzyme for use in the enzymatic treatment, it is preferred to use a restriction enzyme or a nuclease. With regard to the kind of the restriction enzyme, it is also possible to use plural enzymes.
As the mechanical crushing treatment, a method of cleaving the nucleic acid using balls of glass, stainless steel, zirconia, or the like may be mentioned.

The above nucleic acid may be subjected to a treatment by a subtraction method. The subtraction method means a method of efficiently isolating a gene by subtracting a difference existing in the gene in a subtraction manner in the case where the difference is present in the copy number or expression of a target gene. For example, a PCR-Select method, an RDA (representational difference analysis) method, a DsDD (Duplex-specific Direct Digestion) method, and the like can be employed.
Particularly, in the present embodiment, in the case of using a sample nucleic acid derived from a cancer cell, since a large amount of normal cells are contained in a cancer tissue together with the cancer cell, there is a case where performance of nucleic acid microarrey is remarkably decreased but the performance decrease can be prevented to some extent by the subtraction method.

As n kinds of the sample nucleic acids, it is preferred to contain at least plural kinds of analyte nucleic acids and at least one kind, preferably two or more kinds of standard nucleic acids.
Moreover, as another embodiment of the present embodiment, it is possible to use no sample nucleic acid, i.e., all n kinds of sample nucleic acids may be analyte nucleic acids.
The analyte nucleic acid is so-called nucleic acid obtained from an analyte, i.e., a target nucleic acid for which nucleic acid mutation is unknown and the nucleic acid mutation is to be analyzed by the present embodiment. For example, a nucleic acid extracted from a normal cell (non-canceration cell) or a nucleic acid extracted from an abnormal cell (e.g., cancer cell) can be used as an analyte nucleic acid.
The stranded nucleic acid is a nucleic acid for comparison with the analyte nucleic acid in the above and a nucleic acid for which the user (performer of the method for analyzing nucleic acid mutation) has some information on mutation and the like to some extent beforehand. Specifically, with regard to a copy number variation (CNV) or the like, it is preferably a nucleic acid for which the position and the number of the copy number variation are known and is more preferably a nucleic acid whose base sequence is known.
Moreover, for example, when a cancer is generated in a patient having CNV or some mosaic, in the case where a nucleic acid obtained from the cancer cell and a nucleic acid obtained from a normal cell other than the cancer cell for the same patient, the CNV or the some mosaic is offset, so that no problem arises in the use of the nucleic acid obtained from the normal cell as a standard nucleic acid. Therefore, even when the copy number in the genome is different from a generally existing amount in the biological species thereof, the nucleic acid can be used as a standard nucleic acid as long as information can be obtained from the comparison with the analyte nucleic acid, for example, in the case of the same solid.

Each of the above sample nucleic acid is preferably labeled with the same label.
Labeling means that a detectable substance is bound to a nucleic acid. The detectable substance is not particularly limited and there may be mentioned fluorescent substances, enzymes, radioactive isotopes, compounds inducing FRET (fluorescent resonance energy transfer), and the like. A fluorescent label with a fluorescent substance is preferable.

The fluorescent substance is not particularly limited and there may be used fluorescein isothiocyanate (FITC), Cy-dye, Alexa, Green Fluorescent Protein (GFP), Blue Fluorescent Protein (BFP), Yellow Fluorescent Protein (YFP), Red Fluorescent Protein (RFP), Acridine, DAPI, Ethidium bromide, SYBR Green, Texas Red, rare-earth fluorescent labeling agent, TAMRA, ROX, and the like. It is preferred to conduct fluorescent labeling with a Cy-dye such as Cy-3 or Cy-5.
Furthermore, with regard to labeling, Digoxigein (DIG), biotin, and the like can be utilized. As an example of utilizing biotin, when avidin is bound to biotin which has been bound to a probe, an alkaline phosphatase to which biotin has been bound is bound thereto, and nitroblue tetrazolium and 5-bromo-4-chloro-3-indolyl phosphate that are substrates for the alkaline phosphatase are added, purple coloration is observed and thus can be used for detection.
Moreover, labeling can be performed in a non-enzymatic manner. For example, ULS arrayCGH LabelingKit (manufactured by Kreatech Biotechnology BV Company) and the like can be also used.

As a method for fluorescent labeling, either labeling method of a direct labeling method and an indirect labeling method may be used. The direct labeling method means a method where a nucleic acid is transformed into a single-strand one, a short-chain nucleic acid is hybridized thereto, and a nucleotide compound to which a fluorescent substance (e.g., Cy-dye) has been bound is mixed with the nucleotide, thereby the nucleic acid is labeled in one step. The indirect labeling method means a method where a nucleic acid is transformed into a single-strand one, a short-chain nucleic acid is hybridized thereto, a nucleotide compound having a substituent capable of being bound to a fluorescent substance (e.g., Cy-dye), for example, a nucleotide compound having an aminoallyl group and the natural nucleotide are mixed together, a nucleic acid having the substituent is first synthesized, and then a fluorescent substance (e.g., Cy-dye) is bound through the aminoallyl group, thereby the nucleic acid being labeled.

As methods for introducing a labeling compound such as a fluorescent substance into the nucleic acid, a random primer method (primer extension method), a nick translation method, a PCR (Polymerase Chain Reaction) method, a terminal labeling method, and the like may be used. Particularly, in the present embodiment, the random primer method can be suitably used.
The random primer method is a method where a random primer nucleic acid having several bp (base pair) to over ten bp is hybridized and amplification and labeling are simultaneously performed using a polymerase, thereby a labeled nucleic acid being synthesized. The nick translation method is a method where, for example, a double-strand nucleic acid to which nick has been introduced with DnaseI is subjected to the action of a DNA polymerase to decompose DNA and simultaneously synthesize a labeled nucleic acid by the polymerase activity. The PCR method is a method where two kinds of primers are prepared and a PCR reaction is carried out using the primers, thereby amplification and labeling being simultaneously performed to obtain a labeled nucleic acid. The terminal labeling method is a method where, in a method of labeling a 5'-end, a labeling compound such as a fluorescent substance is incorporated into a 5'-end of a nucleic acid dephosphorylated with an alkaline phosphatase by a phosphorylation reaction with a T4 polynucleotide kinase. A method of labeling 3'-end is a method where a labeling compound such as a fluorescent substance is added to a 3'-end of a nucleic acid with a terminal transferase.

As the labeled sample nucleic acid or the like, it is also possible to use an unpurified solution containing the same. In the case of using such an unpurified solution, an enzyme and the like still remain in the solution and hence, after preparation, it is preferable to deactivate the activity of the enzyme remaining in the solution. It is based on the viewpoint of preventing the influence on reproducibility of data.
As methods for deactivating the enzyme, any methods may be possible as long as they can deactivate the enzyme but it is preferable to perform any one or both of a method of adding a chelating agent or a heating treatment at 60°C or higher. The heating temperature is preferably 60°C or higher, more preferably 63°C or higher. The heating time is sufficiently 1 minute or more and most preferably, it is preferred to perform the heating treatment at 65°C or higher for 5 minutes or more.
Moreover, in the case of labeling method using a klenow fragment, it is also possible to deactivate the activity of the enzyme using a vortex mixer or the like.

### Hybridization

In the step (a), in order to bring n kinds of the labeled sample nucleic acids S1 to Sn one by one to each of n sets of the above probe nucleic acid sets, as a method for incorporating the sample nucleic acids one by one into each of the above spots, any methods such as pipetting can be applied.
In the step (a), the probe nucleic acid and the sample nucleic acid can be hybridized by incubating them in a hybridization solution.
The conditions for the incubation are not particularly limited but the incubation is preferably conducted in the range of 25 to 50°C, more preferably conducted in the range of 30 to 45°C, and further preferably conducted in the range of 37 to 42°C. Moreover, the time for the hybridization is preferably 8 to 96 hours, more preferably 12 to 72 hours, and further preferably 16 to 48 hours.

As the hybridization solution for use in the hybridization in the step (a), there is no particular limitation as long as the probe nucleic acid and the sample nucleic acid are hybridized in the solution. It is preferable to be a solution having a nature that the probe nucleic acid and the sample nucleic acid are suitably hybridized and it is more preferable to be a solution having an action to accelerate hybridization having a high degree of coincidence between the sequences of the probe nucleic acid and the sample nucleic acid and, on the other hand, to suppress hybridization having a low degree of coincidence between the sequences.

The hybridization solution preferably contains a substance having an extruded volume effect, a substance for lowering the melting point of the sample nucleic acid, and a solution for adjusting ion intensity.
As the substance having an excluded volume effect, polyethylene glycol (PEG, see, e.g., JP-A-2000-325099), dextran sulfate, and the like may be mentioned.
As the substance for lowering the melting point of the sample nucleic acid, there may be mentioned formamide, glycerol, formaldehyde, dimethyl sulfoxide (DMSO), N,N-dimethylformamide (DMF), guanidine thiocyanate (GuSCN), iodine, and the like.
As the solution for adjusting ion intensity, there may be mentioned SSC (150mM NaCl, 15mM sodium citrate), SSPE (150mM NaCl, 10mM NaH₂PO₄.H₂O, 1mM EDTA pH7.4), MES hybridization buffer, and the like.

Into the hybridization solution, a blocking agent, a surfactant, and the like may be incorporated.
As the surfactant, cationic surfactants, anionic surfactants, nonionic surfactants, and the like may be mentioned. As the anionic surfactants, sodium dodecyl sulfate (SDS) and the like may be mentioned. As the nonionic surfactant, Tween (registered trademark), Triton X (registered trademark), and the like may be mentioned.
The above blocking agent is used for reducing the detection of non-specific hybridization at the hybridization. For example, there may be mentioned tRNA (transfer RNA), modified salmon sperm DNA, poly A (polyadenylic acid), poly dA (polydeoxyadenylic acid), skim milk, and the like, which mainly have an action to reduce background noise of a nucleic acid microarray. Also, generally commercially available blocking agents can be employed.

In addition, in the present embodiment, as described in JP-A-2005-087109, as one composition of the hybridization solution, a phospholipide, a Denhardt's solution (a solution containing ficoll, polyvinylpyrrolidone, and bovine serum albumin as main components), betain that is a quaternary ammonium salt (Biochemistry 32, 137-144 (1993)), TMAC (tetramethyl ammonium chloride) (Proc. Natl. Acad. Sci. USA, 82,1585-1588 (1985)), commercially available hybridization solutions, for example, ExpressHyb (manufactured by Clontech Company), PerfectHyb (manufactured by TOYOBO Co., Ltd.), ULTRAhyb (manufactured by Ambion Company), and the like can be also employed.

For the hybridization, it is preferred to hybridize a target nucleic acid and a probe nucleic acid under stringent conditions. As specific hybridization conditions, for example, after the hybridization reaction is carried out at 37°C for 16 hours, as washing conditions, there may be mentioned washing (1) with 5 mL of 2×SSC solution at room temperature for 30 seconds, (2) with 5 mL of a 50% formaldehyde/2×SSC (pH7.0) solution at 50°C for 15 minutes, (3) with 5 mL of a 2×SSC/0.1% SDS (pH7.0) solution at 50°C for 30 minutes, (4) with 5 mL of a 2xSSC solution at room temperature for 5 minutes.

### Step (b)

In the step (b), label values F1 to Fn of the sample nucleic acid hybridized in n pieces of the spots X1 to Xn were obtained.
In the step (b), the method for obtaining the label intensities after hybridization on the nucleic acid microarray is not particularly limited as long as the label intensities can be measured.
In the step (b), the label intensity of the spot X1 is shown as F1, the label intensity of the spot X2 is shown as F2, ... the label intensity of the spot Xn-1 is shown as Fn-1, and the label intensity of the spot Xn is shown as Fn. n is an integer of 3 or more.
For example, in the case of a radioisotope, X-ray films, BASS (manufactured by FUJIFILM Corporation), and the like can be employed.
In the case where a fluorescent substance is used as a labeling substance, it is preferable to use a fluorescence scanner. As the fluorescence scanner, for example, there may be mentioned FLA series (manufactured by FUJIFILM Corporation), GenePix series (manufactured by Axon Instruments Inc.), LS Reloaded (manufactured by TECAN Company), and the like.

### Step (c)

In the step (c), the label value F1 of the spot X1 is compared with each of the label values F2 to Fn to thereby obtain n-1 pieces of comparison values C2 to Cn and determination is performed whether each of the comparison values falls within a prescribed numerical value range or not. In other words, a temporary determination is performed whether the magnitude of each of the comparison values corresponds positive or not. The positive/negative (particularly determination results of the comparison values with the standard nucleic acid among S2 to Sn) in the conventional methods including no step (d) corresponds to the temporarily determined positive/negative herein.
The comparison value is a numerical value obtained by comparing the label intensities of two spots of the same kind each other and a label intensity ratio, a difference between the label intensities, and the like may be mentioned. The comparison value is preferably the label intensity ratio, more preferably a logarithmic value (Log value) of the label intensity ratio, and further preferably a logarithmic value (Log₂ value) of the label intensity ratio using 2 as a base.
A criterion for temporarily judging each of the comparison values positive or negative, i.e., a prescribed numerical value range may be equal to that of the conventional array comparative genomic hybridization technique and, for example, a sample nucleic acid which is known to be positive (positive control) and a sample nucleic acid which is known to be negative (negative control) are prepared and the judgment can be performed using the intermediate value thereof as a threshold value.

Moreover, since there is a possibility that the amounts of n kinds of the sample nucleic acids to be hybridized are different from one another, it is preferred to include a step for correcting the same. Such a step is not particularly limited and there can be applied normalization usually employed in hybridization of a monochromic method where label intensities obtained in different probe nucleic acid sets are compared. For example, global normalization and the like can be used.
For example, there may be mentioned a method of making the average value, or as in the step (c2) described later, central value of the label intensities in p pieces of the spots in each array even among the arrays to be compared and a method of, after each comparison value in each spot between one pair of arrays is obtained, making the average value or central value of p pieces of the comparison values between the one pair of the arrays equal to that between the other one pair of arrays. Specifically, there may be mentioned methods of representation by ratios obtained from division of individual label intensities or comparison values with the average value or central value of the label intensities in p pieces of the spots in each array, values obtained by subtracting the average value or central value of the label intensity, or deviation values.
By including such a step, the difference induced by the amounts of the sample nucleic acids as mentioned above can be corrected. Here, the method for the correction is not particularly limited and, for example, there may be mentioned methods of multiplication and division of the label intensities with the average value or central value, elimination of values that are deviated from the average value or central value by a certain value or more.

Moreover, in the step of the above correction, in the case of the ratios obtained from division with the average value or central value, the resulting values are values corresponding to the copy number. As the prescribed numerical value range in the aforementioned step (c), it may be possible to set values which can exclude the region where mutation is clearly undergone with reference to each measured value (particularly a value obtained from measurement of known mutation) or it may be possible to set values based on numerical values which are possible as theoretical copy numbers. As the latter, specifically, in the case where the number of a specific nucleic acid region existing on an abnormality-free autosome is 1 copy and the copy number of one pair of the above chromosome pair is 1, theoretically possible copy numbers are values at intervals of 0.5, such as 0, 0.5, 1, 1.5, 2 ... Therefore, as an criterion for performing determination in the above step (c), it is possible to judge the temporary positive or negative using a half of the above interval, i.e., 0.25 as a threshold value or to exclude an intermediate value (e.g., 0.2 to 0.3) without performing judgment as false positive as before.
The prescribed numerical value range of the comparison values can be set similarly to the criterion for judgment as positive in the conventional array comparative genomic hybridization. For example, in the case of using the fluorescent intensity ratio, theoretically, values such as 0.8 to 1.2 can be mentioned but the values can be arbitrarily set in consideration of identification of a sequence or the like. For example, in the case of performing correction to be mentioned later, multiplication or division can be made by such a degree.

Namely, in the present embodiment, the above step (c) is preferably a step of performing the following steps (c1) to (c3) for p pieces of spots existing in each of n sets of the probe nucleic acid sets. Namely, (n-1)×p pieces of comparison values are calculated in total.
[(c1) a step of selecting a pair of two probe nucleic acid sets, which are selected so as to contain the spot X1, from n sets of the above probe nucleic acid sets and
   comparing the label intensities in each of p pairs of spots to which the same probe nucleic acid has been immobilized, between the two probe nucleic acid sets to calculate p pieces of comparison values]
[(c2) a step of calculating an average value or a central value of p pieces of the comparison values obtained in the step (c1)]
[(c3) a step of setting a prescribed numerical value range based on the average value or central value obtained in the step (c2) and determining whether each ofp pieces of the comparison values exceeds the prescribed numerical value range or not]
More specifically, the present embodiment includes the following steps:
n pieces of the same probe nucleic acid sets NS1 to NSn are provided,
each probe nucleic acid set NSi provides a nucleic acid microarray having p pieces of spots tᵢₗ to tᵢₚ, i being an integer and satisfying 1≤i≤n and p being an integer and satisfying 2≤p.
probe nucleic acids different from one another are immobilized on p pieces of the spots tᵢₗ to tᵢₚ of each probe nucleic acid set NSi.
one of p pieces of the spots of each probe nucleic acid set NSi is the spot Xi,
the same probe nucleic acid is immobilized on the spots tᵢⱼ to tₙⱼ, j being an integer and satisfying 1≤j≤p,
the step (a) includes bringing the labeled sample nucleic acid Si into contact with the probe nucleic acid set NSi to hybridize the sample nucleic acid Si to the nucleic acids having been immobilized in p pieces of the spots of the probe nucleic acid set NSi,
the step (b) includes obtaining label values Fᵢⱼ to Fᵢₚ of the sample nucleic acid having been hybridized in the spots tᵢₗ to tᵢₚ of each probe nucleic acid set NSi,
NS1 and NSm are selected from the probe nucleic acid sets NS1 to NSn and m being an integer and satisfying 2≤m≤n,
the step (c) includes the steps:
   (c1) comparing the label values F1j and Fmj to determine p pieces of comparison values C1" to Cp",
   (c2) calculating an average value or a central value of the comparison values C1" to Cp", and
   (c3) setting a prescribed numerical value range based on the average value or central value obtained in the step (c2) and judging whether the comparison values C1" to Cp" fall within the prescribed numerical value range or not.

In the step (c3), it is preferred that the comparison value is a label intensity ratio and the temporary judgment as positive or negative is performed on the basis of deviation of the average value or central value of the label intensity ratios. For example, a threshold value (prescribed numerical value range) is set based on the deviation of the average value or central value and a case where the deviation from the average value or central value exceeds the above threshold value can be temporarily judged positive (or negative).
The step of calculating the average value or central value of the comparison values and the step of comparing it with individual comparison values in the steps (c2) and (c3) can be also carried out, before obtaining the comparison values as mentioned above, by calculating the average value or central value of individual label intensities in p pieces of spots in each probe nucleic acid set and comparing it with individual label intensities. Namely, in the present embodiment, the above step (c) may be the following step:
[(cc) a step of calculating an average value or a central value of label intensities in individual spots in n sets of the probe nucleic acid sets,
calculating deviation values (F'1, F'2 ... F'n) of individual spots based on the average value or central value, in each of n sets of the probe nucleic acid sets,
calculating a comparison value of the label intensities of paired spots of the same kind between a pair of two sets among n sets of the probe nucleic acid sets and determining whether each of n-1 pieces of the comparison values from the comparison value of a pair of F'1 and F'2 to the comparison value of a pair of F'1 and F'n falls within a prescribed numerical value range or not on the basis of the average value or central value of n sets].

Incidentally, in the present embodiment, explanation is conducted with numbering individual steps as different numbers for the sake of explanation but the numbering does not limit the order thereof and also does not exclude simultaneous processing.

### Step (d)

In the step (d), in the case where the number having been determined as not falling within the prescribed numerical value range exceeds a prescribed number among n-1, the spot X1 is decided as positive. The prescribed number is preferably a half of n-1 and cases where n is 4 or more and the prescribed number is 70% of n-1 are more preferable. Moreover, a case where the determined number is the certain number or less is preferably decided as negative but it is also preferable that a certain range is defined and a case falling within the defined range is judged false positive and re-inspection is performed. Even in the case where the re-inspection is performed, it is expected to reduce a possibility of necessity of the re-inspection to a large extent as compared with the conventional method. Moreover, in the case where the re-inspection is performed, it is preferable to perform the re-inspection with changing the comparison subject.
In this regard, in the case where the number of the cases where the comparison values having been determined as not falling within the prescribed numerical value range does not exceed the prescribed number among n-1 in the step (d), the data in the spot X1 may be judged lack of reliability and a processing such as re-inspection can be performed.
Moreover, the spot (X1) is an arbitrary spot and usually, in a plurality of spots (preferably all spots) in the same array which contains the spot X1, steps the same as the steps (a) to (d) of the present embodiment or the treatments (i) to (iii) are performed with replacing the above (X1).

### Correction of Label Intensity

The above label intensity obtained in the step (b) is preferably a corrected value.

Specifically, the label intensity obtained in the step (b) is preferably a value obtained from the following step.

It is preferred to include a step of hybridizing the labeled sample nucleic acid and the immobilized probe nucleic acid in a certain spot,
a step of adding a labeled verifying nucleic acid having a sequence hybridizable to at least a part of the above probe nucleic acid and labeled with a label different from the label of the labeled sample nucleic acid and hybridizing the added nucleic acid to the probe nucleic acid,
a step of obtaining label intensity of the hybridized labeled sample nucleic acid in the above spot,
a step of measuring label intensity of the hybridized labeled verifying nucleic acid in the spot, and
a step of detecting an amount of the immobilized probe nucleic acid in the spot based on the label intensity of the verifying nucleic acid and correcting the label intensity of the sample nucleic acid by the detected amount.

The correction method can use the method described in JP-A-2010-004873, for example.

Here, it is preferred that the step of hybridizing the labeled sample nucleic acid and the step of hybridizing the labeled verifying nucleic acid (B) are simultaneously carried out and
the step of measuring the amount of the hybridized labeled sample nucleic acid and the step of measuring the amount of the hybridized verifying nucleic acid are simultaneously carried out.
Here, the verifying nucleic acid means a nucleic acid having a sequence hybridizable to at least a part of the probe nucleic acid and can be used for detecting variation caused by unevenness of hybridization ability in a plurality of spots, specifically the amount of the probe nucleic acid immobilized in each spot and further for correcting variation in amount of the probe nucleic acid among the spots. Therefore, the verifying nucleic acid is sufficiently a nucleic acid hybridizable to the probe nucleic acid immobilized in all the spots on the nucleic acid microarray and may have any sequence and chain length. Here, all the spots represent all spots having a possibility of reacting the analyte or the standard in the same solution and usually are all the spots on the single nucleic acid microarray but are not limited thereto.
Moreover, the verifying nucleic acid may be any nucleic acid as long as it is hybridizable and may be natural nucleic acids such as DNA and RNA and also nucleic acids obtained by chemical modification of natural nucleic acids such as DNA and RNA.
For example, PNA, methyl phosphonate-type DNA, phosphorothioate-type DNA, chimera-type nucleic acids, and the like can be used.

As a sequence preferable as the verifying nucleic acid, a repetitive sequence may be mentioned. The repetitive sequence is also referred to as a repeat sequence and is a sequence, which repeatedly appears every a certain number of bases, such as a restriction enzyme recognition site. There may be mentioned sequences where a pattern of a short base sequence is repeated many times, for example, poly d(AT) and poly d(GC), and sequences where a long sequence whose one unit may reach several thousand base pairs is repeated many times, and it is known that such a sequence appears in genome with high frequency in higher animals such as human. In the present embodiment, for example, Cot-1 DNA commercially available from Invitrogen Corporation can be suitably used.
Cot-1 DNA is known as a nucleic acid which is used for blocking the repetitive sequence of an analyte obtained from mammalians at nucleic acid microarray measurement.

By labeling at least a part of the Cot-1 DNA and using the labeled one together with unlabeled Cot-1 DNA, it is also possible to enhance detection sensitivity of a specific hybridization and reduce the difference among the spots.

As the verifying nucleic acid, unlike the analyte nucleic acid, it is also preferable to use a nucleic acid which is different from the analyte nucleic acid and has a sequence that all the probe nucleic acid sets commonly have.
For example, it is also possible to use, as the verifying nucleic acid, a synthesized oligonucleotide having a nucleic acid sequence substantially complimentary to the nucleic acid sequence part commonly present in the probe nucleic acids.
Moreover, it is also possible to use, as the verifying nucleic acid, nucleic acids having a sequence that is considered to be present in all the spots upon the preparation of the array, such as a nucleic acids of vectors (plasmid, BAC, YAC, PAC, cosmid, virus, and the like), genome of Escherichia coli, and the like which are used at the preparation of the array.

In the present embodiment, the verifying nucleic acid is preferably used in an amount of 1 or more as a molar ratio to the sample nucleic acid. By using it in a molar ratio of 1 or more, performance of the nucleic acid microarray is improved.

The size of the verifying nucleic acid is preferably 20 bp or more, more preferably 20 bp to 1 Mbp, and further preferably 100 bp to 500 kb.
The amount of the verifying nucleic acid to be used is preferably in the range of 0.5 to 2 molar equivalents to the analyte or standard nucleic acid.

### Automation

All the steps of the method for analyzing nucleic acid mutation according to the present embodiment can be performed manually or the steps can be performed by means of a partially automated machine or all the steps can be performed by means of a fully automated machine.

By automation, workers are relieved from cumbersome works, personnel expenses can be reduced, a difference in results induced by the difference in proficiency can be decreased, and a human error can be prevented.

### Program

The following will explain the program of the present embodiment.

The program of the embodiment is a program for performing data processing in the method for analyzing nucleic acid mutation explained in the above, which performs the following procedures (I) and (II):
[(I) a procedure of comparing whether n-1 pieces of the comparison values from F1 with F2 to F1 with Fn fall within a prescribed numerical value range or not]
[(II) a procedure of judging the spot X1 positive in the case where, among all of n-1 pieces of the comparison values temporarily decided in the above (I), the number of the comparison values having been determined as not falling within the prescribed numerical value range by the procedure (I) are present with exceeding a prescribed number].

The procedure (I) preferably includes the following procedures (i) to (v):
[(i) a procedure of, in p pieces of the spots present in each of n sets of the probe nucleic acid sets,
   selecting a pair of two probe nucleic acid sets to be selected so as to contain the spot X1 from n sets of the probe nucleic acid sets and
   calculating a comparison value of the label intensities of paired spots to which the same probe nucleic acid has been immobilized, between the two probe nucleic acid sets]
[(ii) a procedure of performing the calculation of the comparison value in the procedure (i) on each of p pieces of paired spots in the probe nucleic acid sets],
[(iii) a procedure of calculating an average value or a central value of p pieces of the comparison values obtained in the procedure (ii)], and
[(iv) a procedure of setting a prescribed numerical value range from the average value or central value obtained in the procedure (iii) and comparing whether each of p pieces of the comparison values exceeds the prescribed numerical value or not].

By using the program of the present embodiment, the data processing can be efficiently carried out at the processing of the data obtained by measuring the aforementioned label intensities.
The program is usually provided in the form that it is recorded on a recording medium readable on a computer. The recording medium is not particularly limited as long as it is readable on a computer. The recording media of the present embodiment include both of portable ones and fixed type ones and there may be mentioned compact disks (CD), flexible disks (FD), hard disks, semiconductor memories, and the like.
The program of the present embodiment can be delivered with recording it on the above recording media or may be delivered in the form that it is recorded on a storage device of a computer and is transferred into another computer through a network.

The following will explain the method for diagnosing a disease derived from gene mutation according to the present embodiment.
The method for diagnosing a disease derived from gene mutation according to the embodiment uses the method for analyzing nucleic acid mutation explained in the above. As the above disease to be a target of diagnosis, there may be included all diseases such as cancers, genetic diseases, and infectious diseases for which a nucleic acid may have some information, and the disease is not particularly limited.
In the method for diagnosis according to the embodiment, by applying a nucleic acid extracted from a normal cell (non-canceration cell) as a standard nucleic acid and a nucleic acid extracted from an abnormal cell (e.g., a cell derived from a cancer patient, a genetic disease patient, or an infectious disease patient) as an analyte nucleic acid to the method for analyzing nucleic acid mutation explained in the above, the patient who provides the analyte or a lesion of the cell used as the analyte can be diagnosed without influences of false positive and false negative caused by CNV or the like.

### Examples

The following will explain the invention with reference to Examples. The materials, the reagents, the amount and ratio of the substances, the operations, and the like shown in the following Examples can be appropriately changed unless they deviate from the gist of the invention. Therefore, the scope of the invention is not limited to the following specific examples.
In the following Examples, Cy3-male represents one obtained by labeling male DNA with Cy3. Similarly, Cy5-male represents one obtained by labeling male DNA with Cy5. Moreover, Cy5-Cot-1 DNA and the like similarly represent nucleic acids labeled with respective labeling compounds.

### [Example 1]

### 1. Preparation of labeled sample nucleic acid

Into a microtube were placed 3 µL (0.75 µg) of Human Genomic DNA male (manufactured by Promega Corporation, Catalog No. G152; hereinafter referred to as male genome DNA), 8 µL of water (Distilled Water DNAse, RNase Free, GIBCO), 20 µL of 2.5×Random Primers Solution (Invitrogen Corporation) of a labeling system for array CGH (BioPrime (registered trademark) Array CGH Genomic Labeling System (invitrogen)), followed by a heat treatment at 95°C for 5 minutes using a block incubator and still standing at 37°C for 15 minutes.

Thereto were added 5 µL of 10×dCTP Nucleotide Mix (Invitrogen Corporation) of the above labeling system, 3 µL of Cy3-dCTP Bulk Pack 250 nmol (manufactured by GE Healthcare Bioscience Corporation), 1 µL of Exo-Klenow Fragment (Invitrogen Corporation) of BioPrime (registered trademark) Array CGH Genomic Labeling System, followed by carrying out an amplification reaction simultaneously to a labeling reaction at 37°C for 2 hours using the block incubator. Then, the whole was heated to 65°C using the block incubator to deactivate Exo-Klenow Fragment contained in the reaction solution.

The above operations, genome extraction was carried out from Cell Strain GM07189 (hereinafter referred to as analyte nucleic acid No.1), Cell Strain GM07189 (hereinafter referred to as analyte nucleic acid No.2), Cell Strain GM13451 (hereinafter referred to as analyte nucleic acid No.3), and Cell Strain GM13451 (hereinafter referred to as analyte nucleic acid No.4) of Coriell institute for medical following a protocol of puregene (QIAgen Company). For the extracted DNA, OD was measured using NanoDrop (Scrum Inc.). On the thus obtained genome, the same operations as in the case of the above Human Genomic DNA male were performed to obtain an unpurified labeled sample nucleic acid. Also, for these, Cy3-dCTP (manufactured by GE Healthcare Bioscience Corporation) was used as a labeling compound.

### 2.1. Preparation of labeled verifying nucleic acid

A labeled verifying nucleic acid (unpurified) was obtained in the same manner except that 3 µg (13 µL) of Cot-1 DNA (manufactured by Invitrogen Corporation, Catalog No. 15279-011) was used into a microtube and 3 µL of Cy5-dCTP Bulk Pack 250 nmol (manufactured by GE Healthcare Bioscience Corporation) was used as a labeling compound.

### 2.2 Preparation of hybridization solution containing labeled verifying nucleic acid

Into a microtube were added 21 µL of a labeled nucleic acid Cy5-Cot-1 DNA, 62 µL of unlabeled Cot-1 DNA (62 µg, manufactured by Invitrogen Corporation), 0.3 µg of yeast tRNA, and 9 µL of 3M sodium acetate (pH 5.2), and 360 µL of 99.5% (v/v) ethanol at -20°C was added thereto to effect precipitation with ethanol. After drying, 8.7 µL of water, 6.5 µL of formaldehyde, and 16.8 µL of 20% SDS were added. Thereto were added 32 µL of a dextran-formamide solution obtained by adding water to 1 g of dextran, 5 mL of formamide, and 1 mL of 20xSSC to be a volume of 7 mL, followed by completely dissolving the whole. Thereafter, collection into one tube was conducted and 30 µL each was dispensed into tubes.
Thereto was added 10 µL each of genome of the above labeled sample nucleic acid, and the whole was thoroughly stirred. Then, after incubation at 75°C for 15 minutes, the product was kept at 42°C for 30 minutes or more.

### 3. Preparation of BAC array slide

BAC containing a region that is considered to be a gene region containing congenital abnormality was selected randomly every chromosome from BAC library to which human genome had been inserted and was cultivated in 50 mL of LB medium (+ chloramphenicol) overnight. The above BAC was selected with a certain length every chromosome and finally 340 spots were made.
It was extracted using a plasmid midi kit of QIAGEN Company following the attached protocol. After adjustment to 1.5 µg/16 µLTE, digestion was carried out using restriction enzymes RsaI, DpnI, and HaeIII for 2 hours, respectively, then the enzymes were deactivated at 80°C for 20 minutes, and the three kinds were mixed. Furthermore, an adaptor having a sequence of SEQ No. 1:5'-aattccggcggccgcccgatg-3' and it complimentary strand having a sequence of SEQ No. 2: 5'-P-catcgggcggccgcgg-3 ' (5'-end phosphorylated) were added, the adaptor was attached to the DNA fragments after the restriction enzyme digestion at 95°C for 5 minutes, at 70°C for 15 minutes, at 65°C for 15 minutes, at 60°C for 15 minutes, at 55°C for 15 minutes, at 50°C for 15 minutes, at 45°C for 15 minutes, at 40°C for 15 minutes, at 35°C for 15 minutes, at 30°C for 15 minutes, at 25°C for 15 minutes, and 5 µL of 10×T4 ligase buffer and 2.5 µL of T4 ligase were added to combine the DNA fragments after the restriction enzyme treatment and the adaptor. Then, the DNA fragments were purified using Nucleospin kit (manufactured by MACHEREY-NAGEL Company). PCR was carried out using EX-Taq (manufactured by Takara Bio Inc.) and a 5'-end aminated primer having a sequence of SEQ No. 3: 5'-NH₂-ggaattccgcggccgcccgatg-3'. PCR was carried out 15 cycles as 1st PCR and 10 cycles as 2nd PCR. The product was purified by performing ultrafiltration on a MICROCON column to be a final concentration of 1 µg/µL, which was used as a probe nucleic acid. It was sent to NGK Insulators Ltd. and spotting was carried out on a slide glass of Matsunami Glass Ind., Ltd. by piezo method to form a BAC array.
Furthermore, the BAC array was subjected to blocking and thermal denaturation in accordance with the procedure described in JP-A-2002-176977.

### 4, Hybridization

Hybridization was carried out using HYBRIMASTER HS300 of ALOKA Co., Ltd.
The BAC array slide was placed, temperature was set to be 37°C and, after a hybridization solution was added, hybridization was carried out for 16 hours with stirring. On this occasion, 50% formamide/2×SSC was used as a moisturizing liquid. Thereafter, washing with 5 mL of 2xSSC for 5 minutes and with 10 mL of 2×SSC for 5 minutes at 25°C, washing with 5 mL of 50% formamide/2×SSC for 14 minutes and with 5 mL of 0,1% SDS/2×SSC for 30 minutes at 50°C, and washing with 2xSSC for 5 minutes at 25°C were performed.

### 5. Loading of fluorescent images and data processing

Fluorescent images were loaded using genepix 4000B (manufactured by axon Inc.). Calculation was performed based on gpr files where the images were digitalized. With regard to the calculation method, using cy5 that is a fluorescence value of Cot-1 DNA as an index for correction, correction was carried out on the fluorescence intensity of the labeled genome labeled with cy3 and then comparison was done. As the fluorescence value, one after subtraction of a background fluorescence value was used.
Calculation method: log₂(fluorescence intensity of genome analyte nucleic acid/labeled cot-1 (analyte)/fluorescence intensity of genome standard nucleic acid×labeled cot-1 (standard))
This calculation was performed on all the pairs of all the spots thus hybridized with the analyte nucleic acid No.1 and the same kind of spots in all the spots hybridized with the standard Human Genomic DNA male.
For the values of all the spot pairs obtained by the above calculation, global normalization was performed by division with a central value of all the spot pairs.
Thus, comparison of the analyte nucleic acid No.1 with the standard Human Genomic DNA male was performed. However, since CNV of the standard nucleic acid is not known, there is a possibility that the comparison results may contain false positive and false negative.
Therefore, the comparison with the analyte nucleic acid No.1 was performed using the analyte nucleic acids No. 2, No. 3, and No.4, respectively, instead of the standard Human Genomic DNA male.

### 6. Results

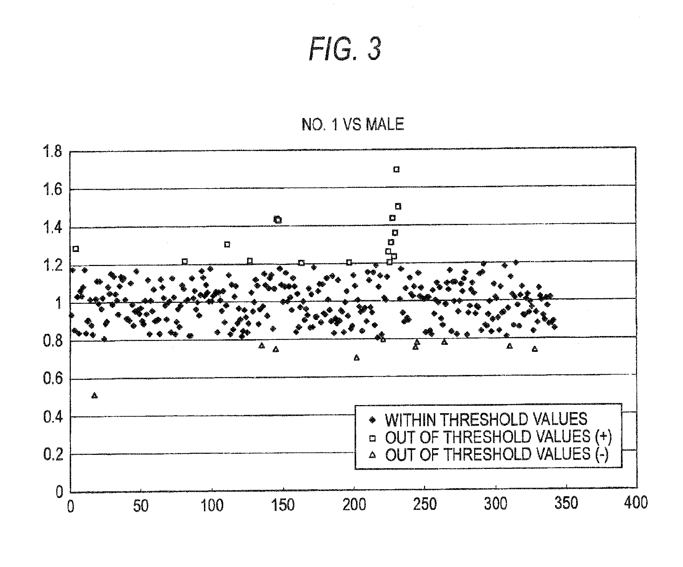

FIG. 3 to FIG. 6 shows results of the above calculation. The axis of ordinate represents a fluorescence intensity ratio and the axis of abscissa represents a spot number (probe nucleic acid number). In FIG. 3 to FIG. 6, ◆ represents data falling within threshold values, □ represents data falling out of the threshold values (ones deviated in a plus direction), and Δ represents data falling out of the threshold values (ones deviated in a minus direction). The threshold values were set to be 0.8 and 1.2.
FIG. 3 is a drawing showing a result of logarithmic plots of fluorescence intensity ratios of analyte DNA No. 1 to male genomic DNA as a standard nucleic acid.

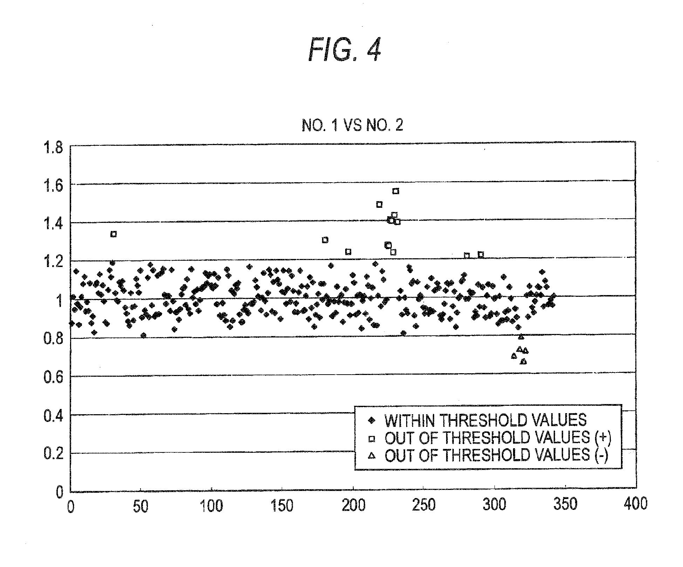

FIG. 4 is a drawing showing a result of logarithmic plots of fluorescence intensity ratios of analyte DNA No. 1 to analyte DNA No. 2.

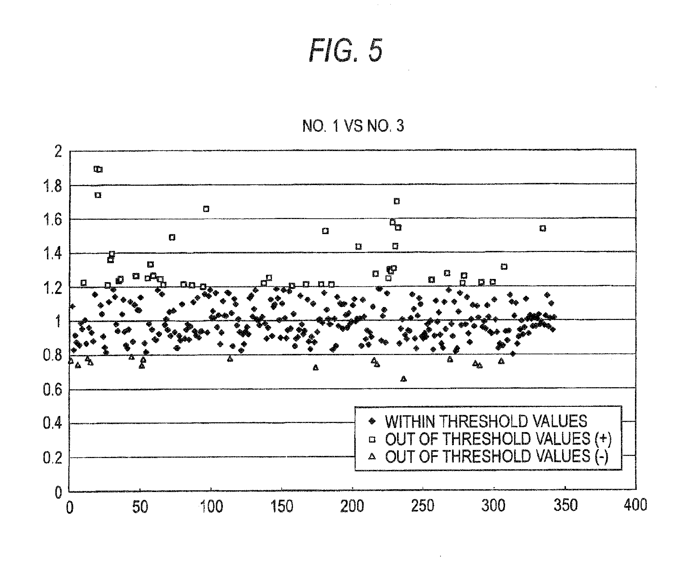

FIG. 5 is a drawing showing a result of logarithmic plots of fluorescence intensity ratios of analyte DNA No. 1 to analyte DNA No. 3.

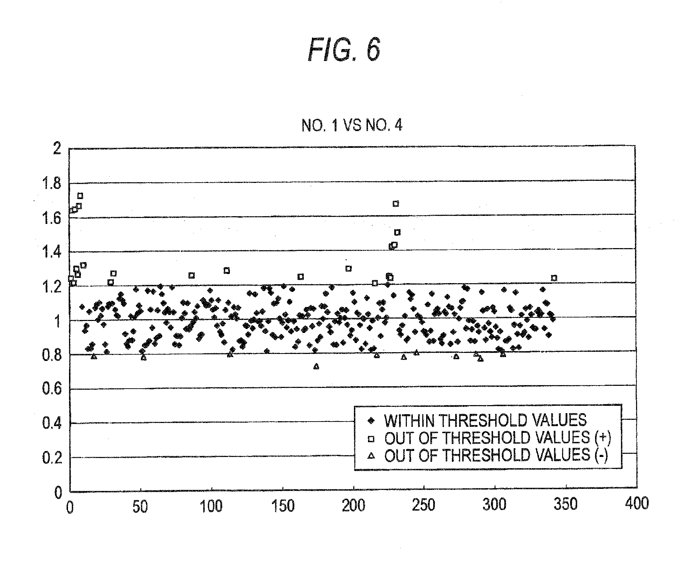

FIG. 6 is a drawing showing a result of logarithmic plots of fluorescence intensity ratios of analyte DNA No. 1 to analyte DNA No. 4.

In each figure, spots which correspond to autosomes and fall out of the threshold values are the following spots.
FIG. 3: 4, 17, 81, 111, 127, 135, 145, 146,147,163, 197, 202, 221, 225, 226, 227, 228, 229, 230, 231, 232, 244, 245, 264, 310, 328
FIG. 4: 31, 181, 197, 219, 225, 226, 227, 228, 229, 230, 231, 232, 281, 291, 314, 318, 319, 320, 321, 322
FIG. 5: 1, 6, 10, 13, 15, 19, 20, 21, 27, 29, 30, 35, 36, 44, 47, 51, 52, 55, 57, 59, 64, 66, 72, 80, 86, 94, 96, 113, 137, 141, 157, 167, 174, 178, 181, 185, 204, 215, 216, 217, 225, 226, 227, 228, 229, 230, 231, 232, 236, 256, 267, 269, 278, 279, 287, 290, 291, 299, 305, 307, 334
FIG. 6: 1, 2, 3, 4, 5, 6, 7, 8, 10, 17, 29, 31, 52, 86, 111, 113, 163, 174, 197, 216, 217, 226, 227, 228, 230, 231, 232, 236, 245, 273, 287, 290, 306, 342
As a result, in each spot of 197 and 225 to 232, the ratio exceeds the threshold values in majority of the results among FIG. 3 to FIG. 6, so that the spots could be judged positive. The other spots exceeding the above threshold values could be judged false positive or false negative.
From the above, the sample of No. 1 was found to have abnormality that the copy number increases in the genes corresponding to the nucleic acids immobilized in the spots 197 and 225 to 232.
Moreover, for the samples of No. 2 to No. 4 and male as a standard, by similar measurement with replacing the above sample of No. 1 therewith, the copy number variation thereof can be measured.
Incidentally, although no example was relevant in the present Example, in the case where both results of a value falling out of the threshold values in a plus direction and a value falling out of the threshold values in a minus direction are present in comparison results of a certain spot between arrays (for example, a case where No. n spot is □ in FIG. 3 and FIG. 4 and No. n spot is Δ in FIG. 5 and FIG. 6), it is preferred not to judge the spot positive.

From the above results, it is realized that positive or negative can be decided by the method of the invention without influences of false positive and the like.

### Industrial Applicability

The method for analyzing nucleic acid mutation, the program, and the method for diagnosis according to the present invention can reduce influences of false positive and false negative in analysis results, particularly, influences owing to CNV of a standard nucleic acid, which are problems in conventional array comparative genome hybridization techniques, and can improve the reliability of the analysis results.

While the invention has been described in detail and with reference to specific embodiments thereof, it will be apparent to one skilled in the art that various changes and modifications can be made therein without departing from the spirit and scope thereof.
The present application is based on Japanese Patent Application No. 2009-209578 filed on September 10, 2009, and the contents are incorporated herein by reference.

## Claims

1. A method for analyzing nucleic acid mutation using array comparative genomic hybridization technique comprising:
(a) a step of, using n sets of the same probe nucleic acid sets where a plurality of spots are present on one nucleic acid microarray and probe nucleic acids different from one another have been immobilized on a plurality of the spots, bringing n kinds of labeled sample nucleic acids S1 to Sn one by one into contact with n sets of the probe nucleic acid sets to effect hybridization,
(b) a step of selecting n pieces of spots X1 to Xn to which the same probe nucleic acid has been immobilized, which are selected from n sets of the probe nucleic acid sets, and obtaining label intensities F1 to Fn of the sample nucleic acid having been hybridized in n pieces of the respective spots X1 to Xn,
(c) a step of comparing the label value F1 of the spot X1 with each of the label values F2 to Fn to thereby obtain n-1 pieces of comparison values C2 to Cn and determining whether each of the comparison values falls within a prescribed numerical value range or not, and
(d) a step of determining whether the number of the comparison values having been determined as not falling within the prescribed numerical value range in the step (c) among n-1 pieces of the comparison values exceeds a prescribed number or not and, in the case of exceeding the prescribed number, judging the spot X1 positive,
n being an integer of 3 or more, the sample nucleic acid being an analyte nucleic acid or a standard nucleic acid, at least the S1 being an analyte nucleic acid, and X1 being a spot with which S1 having been brought into contact.

2. The method for analyzing nucleic acid mutation according to claim 1, wherein at least two kinds among n kinds of the sample nucleic acids are analyte nucleic acids.

3. The method for analyzing nucleic acid mutation according to claim 1 or 2, which further comprises:
(c') a step of determining whether each of n-1 pieces the comparison values from a comparison value of F2 with F1 to a comparison value F2 with Fn falls within a prescribed numerical value range or not and
(d') a step of determining whether the number of the comparison values having been determined as not falling within the prescribed numerical value range in the step (c') among n-1 pieces of the comparison values exceeds a prescribed number or not and, in the case of exceeding the prescribed number, judging the spot X2 positive, the spot X2 being a spot with which a sample nucleic acid S2 different from the above S1 having been brought into contact.

4. The method for analyzing nucleic acid mutation according to any one of claims 1 to 3, wherein at least one kind among n kinds of the sample nucleic acids is a standard nucleic acid.

5. The method for analyzing nucleic acid mutation according to any one of claims 1 to 3, wherein n kinds of the sample nucleic acids are all analyte nucleic acids.

6. The method for analyzing nucleic acid mutation according to any one of claims 1 to 5, wherein n kinds of the sample nucleic acids are nucleic acids derived from different individuals belonging to taxonomically the same species.

7. The method for analyzing nucleic acid mutation according to any one of claims 1 to 6, wherein
n pieces of the same probe nucleic acid sets NS1 to NSn are provided,
each probe nucleic acid set NSi is provided on a nucleic acid microarray having p pieces of spots tᵢₗ to tᵢₚ, i being an integer and satisfying 1≤i≤n and p being an integer and satisfying 2≤p,
probe nucleic acids different from one another are immobilized on p pieces of the spots tᵢₗ to tᵢₚ of each probe nucleic acid set NSi.
one of p pieces of the spots of each probe nucleic acid set NSi is the spot Xi,
the same probe nucleic acid is immobilized on the spots tᵢⱼ to tₙⱼ, j being an integer and satisfying 1≤j≤p,
the step (a) comprises bringing the labeled sample nucleic acid Si into contact with the probe nucleic acid set NSi to hybridize the sample nucleic acid Si to the nucleic acids having been immobilized on p pieces of the spots of the probe nucleic acid set NSi,
the step (b) comprises obtaining label values Fᵢₗ to Fᵢₚ of the sample nucleic acid having been hybridized in the spots tᵢₗ to tᵢₚ of each probe nucleic acid set NSi,
NS1 and NSm are selected from the probe nucleic acid sets NS1 to NSn and m being an integer and satisfying 2≤m≤n,
the step (c) comprises the steps:
(c1) comparing the label values F1j and Fmj to determine p pieces of comparison values C1" to Cp",
(c2) calculating an average value or a central value of the comparison values C1" to Cp", and
(c3) setting a prescribed numerical value range based on the average value or central value obtained in the step (c2) and judging whether the comparison values C1" to Cp" fall within the prescribed numerical value range or not.

8. The method for analyzing nucleic acid mutation according to any one of claims 1 to 7, wherein the sample nucleic acids are all labeled with the same label.

9. The method for analyzing nucleic acid mutation according to any one of claims 1 to 8, wherein the label intensities F1 to Fn in the step (b) are corrected values.

10. A program for performing data processing in the method for analyzing nucleic acid mutation according to any one of claims 1 to 9, which performs the following procedures (I) and (II):
[(I) a procedure of comparing whether n-1 pieces of comparison values from F1 with F2 to F1 with Fn fall within a prescribed numerical value range or not]
[(II) a procedure of judging the spot X1 positive in the case where, among all of n-1 pieces of the comparison values temporarily decided in the above (I), the number of the comparison values having been determined as not falling within the prescribed numerical value range by the procedure (I) are present with exceeding a prescribed number].

11. The program according to claim 10, wherein the procedure (i) includes the following procedures (i) to (v):
[(i) a procedure of, in p pieces of the spots present in each of n sets of the probe nucleic acid sets,
selecting a pair of two probe nucleic acid sets to be selected so as to contain the spot X1 from n sets of the probe nucleic acid sets and
calculating a comparison value of the label intensities of paired spots to which the same probe nucleic acid has been immobilized, between the two probe nucleic acid sets]
[(ii) a procedure of performing the calculation of the comparison value in the procedure (i) on each of p pieces of paired spots in the probe nucleic acid sets],
[(iii) a procedure of calculating an average value or a central value ofp pieces of the comparison values obtained in the procedure (ii)], and
[(iv) a procedure of setting a prescribed numerical value range from the average value or central value obtained in the procedure (iii) and comparing whether each of p pieces of the comparison values exceeds the prescribed numerical value or not]

12. A method for diagnosing a disease derived from gene mutation, which uses the method for analyzing nucleic acid mutation according to any one of claims 1 to 9.


SEQUENCE LISTING

<110>  FUJIFILM Corporation

<120>  Method for analysing nucleic acid mutation using array comparative
genomic hybridization

<130>  10F03498

<160>  3

<170>  PatentIn version 3.1

<210>  1
<211>  21
<212>  DNA
<213>  Artificial

<220>
<223>  synthetic DNA

<400>  1
aattccggcg gccgcccgat g                                                21


<210>  2
<211>  16
<212>  DNA
<213>  Artificial

<220>
<223>  synthetic DNA

<400>  2
catcgggcgg ccgcgg                                                      16


<210>  3
<211>  22
<212>  DNA
<213>  Artificial

<220>
<223>  synthetic DNA

<400>  3
ggaattccgc ggccgcccga tg                                               22